# EUROPEAN PATENT SPECIFICATION

(11) **EP 3 062 685 B1**
(45) Date of publication and mention of the grant of the patent: **31.05.2017**
(21) Application number: 14792495.5
(22) Date of filing: 31.10.2014
(51) Int. Cl.: A61B 5/00, A61B 5/1455

(54) **THERAPY SYSTEM WITH A PATIENT INTERFACE FOR OBTAINING A VITAL STATE OF A PATIENT**
THERAPIESYSTEM MIT EINER PATIENTENSCHNITTSTELLE FÜR DEN ERHALT EINES VITALZUSTANDS EINES PATIENTEN
SYSTÈME THÉRAPEUTIQUE AVEC INTERFACE PATIENT POUR OBTENIR UN ÉTAT VITAL D'UN PATIENT

(30) Priority: 01.11.2013 EP 13191295; 26.03.2014 EP 14161714
(43) Date of publication of application: 07.09.2016
(73) Proprietor: Koninklijke Philips N.V., 5656 AE Eindhoven (NL)
(72) Inventor: LAWRENSON, Matthew John, NL-5656 AE Eindhoven (NL); NOLAN, Julian Charles, NL-5656 AE Eindhoven (NL)
(74) Representative: Freeke, Arnold
(86) International application number: PCT/EP2014/073409
(87) International publication number: WO 2015/063247

(56) References cited:
- WO-A1-2012/127370
- DE-A1-102010 056 478
- US-B1- 8 545 416
- VERKRUYSSE ET AL.: "Remote plethysmographic imaging using ambient light", OPTICS EXPRESS, vol. 16, no. 26, 22 December 2008 (2008-12-22), pages 21434-21445, XP007913060, cited in the application

## Description

### FIELD OF THE INVENTION

The present invention relates to a therapy system with a patient interface for delivering a flow of breathable gas to a patient, wherein the patient interface enables to remotely obtain a vital state of a patient. The present invention further relates to a therapy device that may be used in such a therapy system and to a method for remotely obtaining information on a vital state of a patient.

### BACKGROUND OF THE INVENTION

Patient interfaces, such as masks for covering the mouth and/or nose, are used for delivering gas to a patient. Such gases, like air, cleaned air, oxygen, or any modification of the latter, are submitted to the patient via the patient interface in a pressurized or unpressurized way.

For several chronic disorders and diseases, a long-term attachment of such a patient interface to a patient is necessary or at least advisable.

One non-limiting example for such a disease is obstructive sleep apnea or obstructive sleep apnea syndrome (OSA). OSA is usually caused by an obstruction of the upper airway. It is characterized by repetitive pauses in breathing during sleep and is usually associated with a reduction in blood oxygen saturation. These pauses in breathing, called apneas, typically last 20 to 40 seconds. The obstruction of the upper airway is usually caused by a reduced muscle tonus of the body that occurs during sleep. The human airway is composed of walls of soft tissue which can collapse and thereby obstruct breathing during sleep. Tongue tissue moves towards the back of the throat during sleep and thereby blocks the air passages. OSA is therefore commonly accompanied with snoring.

Different invasive and non-invasive treatments for OSA are known. One of the most powerful non-invasive treatments is the usage of Continuous Positive Airway Pressure (CPAP) or Bi-Positive Airway Pressure (BiPAP) in which a patient interface is connected to a pressure generator via a patient circuit including one or more tubes, wherein the pressure generator blows pressurized gas into the patient interface and into the patient's airway in order to keep it open. Positive air pressure is thus provided to a patient by means of the patient interface that is worn by the patient typically during sleep.

Examples for such patient interfaces are:
- nasal masks, which fit over the nose and deliver gas through the nasal passages,
- oral masks, which fit over the mouth and deliver gas through the mouth,
- full-face masks, which fit over both the nose and the mouth and deliver gas to both, and
- nasal pillows, which are regarded as patient interfaces as well within the scope of the present invention and which consist of small nasal inserts that deliver gas directly to the nasal passages.

One important issue in the treatment of OSA and other diseases is the continuous monitoring of the vital state of a patient. On the one hand, it is possible to adjust or modify the provided therapy based on the current vital state of the patient. On the other hand, the motivation of the patient to comply with a prescribed therapy can often be improved by providing him with feedback on the effect of the therapy on his vital state. In particular in the field of OSA treatment, the compliance of patients is often a problem.

In US 8,545,416 B1 an integrated sleep diagnosis and treatment device is presented. The sleep disorder treatment system presented therein uses a diagnosis device to perform various forms of analysis to determine or diagnose a subject's sleeping disorder or symptoms of a subject's sleep disorder. This analysis or diagnosis can be used to treat the subject either physically or chemically to improve the sleeping disorder or the symptoms of the sleeping disorder. The diagnostic part of the system can make use of different types of sensors and methods for diagnosing the severity of the symptoms of or the sleep disorder itself. The treatment part of the system can use a device to physically or chemically treat the subject's symptoms or sleep disorder itself.

In WO 2012/127370 A1 systems and methods for utilizing blood oxygenation information with respiratory therapy are disclosed. These systems and methods may provide respiratory therapy to a patient, which may include providing a flow of gas to a patient via a patient interface. Blood oxygenation information for the patient is obtained and used to adjust the respiratory therapy, advance a diagnosis for the patient, or for other purposes.

In DE 10 2010 056 478 A1 an apparatus for complex long-term monitoring of human autonomic regulation during sleep or falling asleep relaxation phases is disclosed. The apparatus collects and analyzes the systemic coordination of interaction between heart and respiratory rates. A nasal mask produces breathing air over pressure-generation. A forehead pad of mask is integrated with sensor unit for non-invasive registration of the vegetative parameters such as heart rate, respiratory rate, oxygen saturation and dermal head movement in the forehead skin.

However, there is still a need for further improving the monitoring of a vital state of a patient while being under treatment, in particular of a patient being under OSA treatment by means of a patient interface.

### SUMMARY OF THE INVENTION

It is an object of the present invention to provide a therapy system with a patient interface for delivering a flow of breathable gas to a patient, which allows obtaining information on a vital state of the patient while the patient is under treatment. It is further an object of the present invention to provide a therapy device that is suitable for use in such a therapy system. It is yet another object of the present invention to provide a method for obtaining information on a vital state of a patient.

In a first aspect of the present invention, a therapy system is provided that comprises:
a patient interface for delivering a flow of breathable gas to a patient, wherein the patient interface comprises a detection unit for detecting reflected light from a skin area of the patient;
a processing unit for processing the image signal; and
an evaluation unit for deriving information on a vital state of the patient based on an evaluation of a development of the image signal over time; characterized in that the detection unit (24) is configured to generate an image signal from the detected light and in that the processing unit is configured to identify a blood vessel of the patient within the skin area and to determine a size measure for the blood vessel, and wherein the evaluation unit is configured to evaluate a development of the determined size measure over time to derive information on the vital state of the patient.

In a further aspect of the present invention, there is presented a therapy device comprising:
a pressure generator for generating a pressurized flow of breathable gas for delivery to a patient interface;
a data interface for receiving an image signal from a detection unit that is configured to detect reflected light from a skin area of the patient and to generate the image signal from the detected light;
a processing unit for processing the image signal; and
an evaluation unit for deriving information on a vital state of the patient based on an evaluation of a development of the image signal over time, and wherein the processing unit is configured to identify a blood vessel of the patient within the skin area and to determine a size measure for the blood vessel, and wherein the evaluation unit is configured to evaluate a development of the determined size measure over time to derive information on the vital state of the patient.

In yet another aspect of the present invention, there is presented a method for obtaining information on a vital state of a patient, wherein the method comprises the steps of:
detecting reflected light from a skin area of the patient and generating an image signal from the detected light by means of a detection unit that is comprised in a patient interface for delivering a flow of breathable gas to the patient;
processing the image signal; and
evaluating the image signal for deriving information on the vital state of the patient based on an evaluation of a development of the image signal over time; wherein
the processing includes identifying a blood vessel of the patient within the skin area and determining a size measure for the blood vessel, and wherein the evaluating includes evaluating a development of the determined size measure over time.

In yet further aspects of the present invention, there are provided a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed patient interface, the claimed therapy device and the claimed method have similar and/or identical preferred embodiments as the claimed therapy system and as defined in the dependent claims. The claimed patient interface and the claimed therapy device may be realized as separate entities that are included in the herein presented therapy system.

The continuous monitoring of the vital state of a patient receiving a treatment by means of a patient interface can be of interest for the patient himself or for a physician. Thereby, the vital state can refer to vital signs of the patient, such as the heart rate, the blood oxygen saturation, the breathing rate, the blood pressure, the blood flow rate and to other parameters such as an apnea-hypopnea index (AHI), a sleep quality index, etc.

The present invention allows deriving information on a vital state of the patient from an image signal. For this, there is provided a detection unit, which captures reflected light from a skin area of the patient and generates an image signal based thereupon. This detection unit preferably includes an image sensor providing a signal including information on brightness values of different pixels. This detection unit is mounted to the patient interface and aims at a skin area of the patient. Based on the generated image signal, images of the skin area of interest can be reconstructed by means of a processing unit. An evaluation unit then derives information on the vital state of the patient based on an evaluation of a development of the image signal over time, i.e. either based on a direct evaluation of the image signal itself over time or based on an evaluation of the development of the image sequence of the skin area of interest that may be reconstructed from the image signal. The processing unit and/or the evaluation unit can be arranged at or within the patient interface, but may also be arranged in a separate entity, such as a mobile device, a computer or in a separate therapy device that includes a pressure generator for generating the pressurized flow of breathable gas which is delivered to the patient interface. The processing unit and/or the evaluation unit may in all aforementioned cases be connected to the detection unit of the patient interface by means of a wireless or hard-wired data connection.

One way of measuring vital signs is plethysmography. Plethysmography generally refers to the measurement of volume changes of an organ or a body part and in particular to the detection of volume changes due to a cardio-vascular pulse wave traveling through the body of a subject with every heartbeat. Photoplethysmography (PPG) is an optical measurement technique that evaluates a time-variant change of light reflectance or transmission of a skin area of interest. PPG is based on the principle that blood absorbs more light than surrounding tissue, so variations in blood volume with every heartbeat affect transmission or reflectance correspondingly. Besides information about the heart rate, a PPG waveform (also referred to as PPG signal) can comprise information attributable to further physiological phenomena such as the respiration (breathing rate). By evaluating the transmissivity and/or reflectivity at different wavelengths (typically red and infrared), the blood oxygen saturation can be determined.

Conventional pulse oximeters are often attached to the skin of the subject. Therefore, they are referred to as 'contact' PPG devices. Recently, non-contact, remote PPG (RPPG) devices for unobtrusive measurements have been introduced. Remote PPG utilizes light sources or, in general radiation sources, disposed remotely from the subject of interest. Similarly, also a detector, e.g. a camera or a photo detector, can be disposed remotely from the subject of interest to capture an area of interest of the subject. Therefore, remote PPG systems and devices are considered unobtrusive and well suited for medical as well as non-medical everyday applications. Verkruysse et al., "Remote plethysmographic imaging using ambient light", Optics Express, 16(26), 22 December 2008, pp. 21434-21445 demonstrate that photoplethysmographic signals can be measured remotely using ambient light and a conventional consumer level video camera. A product using the RPPG technique is sold by the applicant as Philips Vital Signs Camera. These types of cameras are usually positioned at fixed locations with respect to the patient. However, subject motion can make it more difficult to extract vital sign information by means of RPPG. A robust vital sign monitoring by means of a fixed camera is often impeded if the monitored subject moves within the field of view of the camera during the measurement procedure.

According to the present invention such a vital sign camera may be mounted to the patient interface and used as the herein called detection unit. This provides the advantage that the detection unit has a more or less fixed position relative to the patient's face. If the patient moves his face, the patient interface will follow this movement, such that the detection unit will usually be pointed at the same skin area. Thereby, a robust vital sign extraction can be achieved.

Thus, the present invention allows deriving information on a vital state of the patient without requiring the patient to carry out a dedicated measurement procedure during his sleep cycle. The patient is only required to wear his mask (patient interface) as usually and the vital state monitoring is carried out automatically without requiring any intervention. Thereby, it becomes possible to monitor a vital state of a patient by means of sensor equipment integrated in a patient interface. In comparison to previous approaches that include dedicated sensors attached to different body parts of the patient, the patient interface according to the present invention provides a higher comfort and usability. It is advantageous that only one device needs to be used and no further sensors (breast belts, finger clips, ear clips, etc.) are required.

However, it shall be noted that the present invention is not restricted to a patient interface with a vital signs camera such as the Philips Vital Signs Camera. The present invention may also make use of a regular image sensor, such as a CMOS sensor, that is implemented and used as the herein called detection unit.

According to an embodiment, the processing unit is configured to reconstruct an image sequence based on the image signal, and the evaluation unit is configured to derive the information on the vital state of the patient based on an evaluation of a development of the image sequence over time.

According to a further embodiment, the evaluation unit is configured to identify a specific area of the face of the patient within the image sequence and to evaluate a color of image pixels of the image sequence in said area in order to derive the information on the vital state of the patient. The evaluation unit is in this case preferably configured to evaluate a red color content of the image pixels of the image sequence in said area and to evaluate a development of said red color content of the image pixels of the image sequence in said area over time in order to derive the information in the vital state of the patient. For example, it is possible to examine over time the average 'redness', or the minimum and/or maximum of the red color content, or a percentage of the red color content over a certain level of red value. In particular by evaluating over time the a percentage of the red color content over a certain level of red value of the pixels in the area of interest, the evaluation unit may be configured to derive therefrom an estimate about the heart rate, a change of rate of flow of blood over time and/or a change of blood pressure over time. Such an estimate may be based on the consideration that an increase in the red color content over time may result from an increased blood flow and/or blood pressure over time. By evaluating the change of the red color content over time, the pulsation of blood may also be seen, such that the heart rate may be extracted.

According to the present invention, the processing unit is configured to identify a blood vessel of the patient within the skin area and to determine a size measure for the blood vessel, wherein the evaluation unit is configured to evaluate a development of the determined size measure over time to derive information on the vital state of the patient.

The processing unit thereto reconstructs an image sequence of the skin area from the image signal and then identifies one or more blood vessels of interest within at least one image of the image sequence. The blood vessel identification within the at least one image of the image sequence may be performed in various ways. According to one embodiment, the processing unit may be configured to identify the blood vessels of interest by means of a landmark detection in the at least one image of the image sequence. This landmark detection could include the identification of distinctive, easy-to-detect landmarks within the face of the patient, such as e.g. the nose of the patient, and then making use of the knowledge how the blood vessels of interest are usually positioned with respect to these landmarks. However, the blood vessels may also be directly detected in the image by an algorithm that detects pixels in the image with high brightness gradients over time, which is typical for such pulsating blood vessels. A still further possibility for identifying the blood vessels within the image is an edge detection algorithm, such as Canny edge detection algorithm.

As soon as the one or more blood vessels are identified within the image sequence, the size changes of these blood vessels may be evaluated over time within the evaluation unit in order to derive information on the vital state of the patient, e.g. in order to derive the blood pressure or a change of the blood pressure over time, as this will become more apparent from the following description.

The size of a blood vessel changes with time (i.e. the blood vessel expands and contracts) during a heartbeat cycle (i.e. at various stages of a heartbeat) of a patient, but also during a longer time interval. The size measure determined by the evaluation unit thus preferably includes a parameter describing a width/diameter of the blood vessel. The changes in the diameter of the blood vessel are evaluated in the evaluation unit. In order to provide meaningful results over a longer time period, it is required that a blood vessel is identified in order to compare it to itself at a previous point in time (i.e. to determine a relative size measure). Based on this development of the determined size measure over time, information on the vital state of the patient may be derived.

According to an embodiment, the processing unit is configured to derive from the image signal an image sequence including at least one image of the skin area, wherein the therapy system further comprises a storage unit for storing previously recorded image sequences of the skin area, and wherein the evaluation unit is configured to derive the information on the vital state of the patient by comparing the at least one image of the skin area with at least one image of the previously recorded image sequences of the skin area.

It is particularly preferred that the evaluation unit thereto compares the width/diameter of the identified one or more blood vessels in the at least one (current) image of the skin area with the width/diameter of the identified one or more blood vessels in the at least one image of the previously recorded image sequences of the skin area. This allows measuring the changes of the width/diameter of a blood vessel over time. This comparison could either include a comparison of the width of the blood vessel in at least two different timely successive stages during the heart cycle or it could include a comparison of the width of the blood vessel at two different instants of time during the same stage of the heart cycle, e.g. when the blood vessel has its maximum or minimum diameter. The latter mentioned two different instants of time could be in different or the same sleep periods, so that a width/diameter change of the blood vessel could be compared on a long-term basis over several days and several sleep sessions always in the same sleep period or between different sleep periods in the same night. This can be used to assess whether the rate of blood flow and/or the blood pressure changes over time.

The present invention may thus replace measurement procedures, such as the determination of a blood pressure change of the patient by means of a sphygmomanometer that includes an inflatable band that goes around the patient's arm and a manometer. The use of such a device usually results in the patient waking up during the measurement procedure, which causes a poorer sleep quality. In comparison to other monitoring approaches the present invention therefore provides higher comfort for the patient.

The information on the vital state of the patient provided by the present invention can, e.g., be used as an educative tool that links patient lifestyle and apnea occurrence frequency and provides this information to the patient. For instance, such information could encourage the patient to modify his lifestyle and/or increase his compliance with a prescribed therapy. Another example for an application area for the present invention could be as part of a system wherein the derived information on the vital state of a patient is used to automatically adjust the settings of a therapeutic device, in particular a PAP machine, which is used for treating the patient.

According to a preferred embodiment, the processing unit is configured to amplify a signal portion of the image signal being indicative of at least one of a movement and a change in color in the skin area.

Such an amplification allows an easier detection of the blood vessels of interest within the reconstructed images, since the color change caused by the blood vessels and/or the movement of the blood vessels is thereby so-to-say artificially exaggerated. An image signal usually includes time-varying brightness values for a plurality of pixels, each pixel usually representing a specific color (e.g. red, green or blue) and position. Some of these pixels (i.e. a signal portion) represent parts of an image that show a movement (e.g. the movement of a pulsating blood vessel) or a color change (e.g. caused by pulsating blood). In order to extract a vital state of the patient, this movement or color change can be amplified to allow a better evaluation. A signal portion may particularly refer to the values of a subset of pixels. Determining said subset may include performing a frequency analysis and/or techniques like independent component analysis (ICA) and the like in order to identify an image portion that shows a movement or color change. The determined signal portion is then amplified. Amplification particularly refers to (artificially) increasing the amplitude of changes in this signal portion. In particular, changes over time are emphasized or magnified. This has the advantage that the further processing in the processing unit may be carried out more efficiently. If the signal (or signal portion) is amplified, the accuracy of the identification of the blood vessel or of the determined size measure may be improved. If the signal is amplified, changes in the size measure become more visible. Thus, also small variations in the size of the blood vessel can be observed. The determined size measure shows an increased variation. Then, the image signal allows a better monitoring of changes of the size measure over time.

In a preferred embodiment, the processing unit is configured to apply Eulerian video magnification to the image signal.

Eulerian video magnification refers to a technique developed by scientists at MIT (Wu et al., "Eulerian Video Magnification for Revealing Subtle Changes in the World", ACM Transactions on Graphics (TOG), Vol. 31, issue 4, July 2012). This technique allows amplifying a signal portion of an image signal. Therefore, a special decomposition is applied to a video sequence followed by a temporal filtering of the video frames. The resulting signal is then magnified with the effect of revealing small color changes and motions that otherwise cannot be seem by the human eye and may be too small for being extracted. Thus, as lined out above, such an amplification or magnification allows an improved determination of a size measure for a blood vessel from the image signal. A blood vessel is usually subject to movements caused by the pulsating blood. This pulsating blood causes the blood vessel to change its size during a heartbeat of the patient. This is monitored in form of the size measure. The application of Eulerian video magnification to the image signal allows making also small changes in the size of the blood vessel visible. Thus, a size measure can be extracted more reliably (compared to a size measure determined based on the original image signal). Even if the original image signal does not reveal the movement or change in color and determine a size measure based thereupon, this may be possible based on the magnified image signal. As the determined size measure based on the magnified image signal will usually not anymore correspond to the original value, it is particularly useful to derive a relative size measure.

The evaluation unit is preferably configured to derive information on the vital state of the patient including at least one vital parameter being indicative of:
- a minimum, maximum or average diameter of the blood vessel during a heartbeat of the patient;
- a blood pressure of the patient;
- a heart rate of the patient;
- an apnea-hypopnea index of the patient;
- a rate of flow of blood of the patient; and
- a variation of the rate of flow of blood.

Thus, the development of the size measure of the blood vessel is analyzed over one heartbeat cycle (or a small number of heartbeat cycles) of the patient and information on the vital state of the patient is extracted therefrom. In this information, a plurality of vital parameters can be included. For instance, a minimum, maximum or average diameter (i.e. a width) of the blood vessel may indicate how the blood vessel changes its diameter during a heartbeat cycle of the patient, which may be used as an indication of the blood pressure of the patient, in particular of an occurring change in blood pressure. Furthermore, by evaluating a plurality of heartbeat cycles, a heart rate of the patient can be extracted. For this, the temporal frequency of occurring maxima or minima in the dimensions of the blood vessel is preferably evaluated, e.g. by means of a Fourier analysis. Still further, an AHI of the patient can be extracted. An apnea or hypopnea event usually has an effect on the blood pressure because a natural response to the drop in blood oxygenation is to constrict the blood vessels in order to prioritize the flow of blood to the heart and brain. These changes in the blood vessel parameter can be monitored and the AHI (or a parameter indicative of the AHI) can be extracted. Still further, a rate of flow of blood can be extracted also based on the development of the diameter of the blood vessel during a heartbeat cycle or during multiple heartbeat cycles. It is also possible to analyze a variation of the rate of flow of blood.

Herein, a vital parameter particularly refers to a parameter extracted from one heartbeat cycle or a small number of heartbeat cycles during a single capturing session. Thus, the detection unit captures reflected light for a plurality of heartbeat cycles and vital parameter is extracted based on the generated image signal. Thereby, it is particularly advantageous that all parameters can be extracted without requiring the patient to perform any specific measurement procedure or the like. Usually, the determined vital parameter will be indicative of the above-described measures on an absolute or relative scale.

According to an embodiment, the evaluation unit is further configured to derive information on the vital state of the patient including at least one trend parameter being indicative of a development of a vital parameter during a complete sleep cycle and/or during a period including multiple sleep cycles of the patient.

In contrast to the vital parameters, such a trend parameter thus refers to a parameter which represents a trend or a long term trend in the development of one of the vital parameters during a time period. For instance, the development of one of the vital parameters during a sleep period of the patient lasting for some hours (sometimes also referred to as a sleep cycle) can be evaluated. Furthermore, it is also possible to analyze how a vital parameter changes from one sleep cycle to another. Thereby, it may be possible to determine an average and/or extreme value of one of the parameters or to consider statistical variation measures etc.. For instance, an apnea-hypopnea index of a patient will usually be extracted for one sleep cycle of the patient in order to provide a meaningful figure. Then, it is possible to analyze how this apnea-hypopnea index changes during a period of time including multiple sleep cycles (e.g. one week).

It is furthermore important to examine always the same part of the face of the patient in order to be able to compare different measurement results with each other. In other words, it should be ensured that the measurement basis always remains the same. The following embodiments account for this problem.

In an embodiment, the processing unit is configured to track the skin area within the image sequence and/or the previously recorded image sequences by applying an image matching algorithm.

This ensures that always the same skin area of interest is examined by the detection unit. If the patient interface is placed in a slightly different position each time it is used, this becomes particularly important. The patient interface may move relative to the face of the patient, e.g. due to the patient moving in his sleep and/or interfering with an external object (such as his pillow). In such cases, the detection unit can be unintentionally moved relative the skin of the patient and it becomes necessary to retrieve the same blood vessels as before in the skin area in order to determine the size measure. Image matching algorithms may thereto be applied. In other words, the currently taken images may be compared to previously taken images in which the position of the skin area of interest is known. Such image matching algorithms may also include landmark detection. In particular, facial features, such as the nose or the eyes of the patient can be detected and the blood vessel can be identified based on its relative position to them. Other techniques may include the detection of differently colored areas or characteristic skin portions such as birth marks etc.. One advantage of the application of image matching algorithms is that it becomes possible to monitor the same skin area even though the patient interface has been moved relative to the face of the patient. If the field of view of the detection unit (image sensor) is large enough, a re-arrangement is not even necessary for this skin area tracking.

In a further embodiment, the patient interface may comprise an orientation sensor for measuring an orientation of the detection unit relative to the skin area of the patient.

Such an orientation sensor may include a proximity sensor which provides a measure for the distance between the detection unit and the skin area of the patient, or an inertial sensor, such as an acceleration sensor or a gyroscope sensor for providing an absolute or relative orientation of the detection unit.

The measurement of a proximity sensor may be used to electronically compensate the measurements for changes in the distance between the patient interface and the patient's face. The proximity and/or inertial sensor may also support the above-mentioned identification/tracking of the skin area. Alternatively, the patient interface could also comprise a visual, audible or tactile output unit that is connected to the orientation sensor and configured to output a warning signal to the user as soon an orientation and/or position change is detected. This warning signal may indicate the user to re-position his patient interface in order to bring it back into its original/optimal position.

In a still further embodiment, the patient interface may further comprise an actuator for adjusting a position and/or alignment of the detection unit relative to the skin area of the patient based on the orientation measured by the orientation sensor.

Such an actuator may include an electric motor or other actuator that allows influencing the orientation of the detection unit relative to the skin area of the patient. The skin area of interest may thus be tracked in an easier way. For instance, it may be advantageous to configure the actuator to always keep the detection unit in a parallel alignment relative to the skin area of interest. Such a parallel alignment may help to obtain a more reliable identification of a blood vessel. Also the determination of the size measure for the blood vessel may be improved. The effect of a parallel alignment is that it is not required to consider artifacts in the image that result from perspective displacements. It may be also advantageous to provide a constant distance between the detection unit and the skin area of interest. This may be achieved by means of an actuator for providing a movement of the detection unit perpendicular to the skin area.

In another embodiment, the therapy system further comprises a temperature sensor for measuring a body temperature and/or an ambient temperature, wherein the evaluation unit is configured to correct the evaluation of the development of the image signal based on the body temperature and/or the ambient temperature.

In other words, the evaluation unit is in this embodiment configured to calibrate the determined size measure for the blood vessel based on a body temperature of the patient and/or an ambient temperature. One important factor influencing the size of the blood vessel of a patient is the body temperature of the patient. Usually, a higher body temperature will result in larger blood vessels. This can, e.g., be caused by an increased ambient temperature or other effects. This effect of the body temperature, however, is not indicative of a vital state of the patient but merely represents an artifact when trying to derive information on a vital state of the patient by means of the system presented herein. Therefore, it is advantageous to calibrate the size measure based on a body temperature of the patient. Such a body temperature may, e.g., be measured by means of an external temperature sensor that is also integrated in the patient interface or also by a remote sensor providing a temperature signal to the evaluation unit. The calibration may, e.g., be carried out by means of a mathematical calibration function or by means of a look-up table. Preferably, the evaluation unit is configured to calibrate the determined size measure for the blood vessel based on a calibration value from a look-up table including predetermined calibration values for different body temperatures. If a calibration data set is available that includes calibration values for different body temperatures of the patient in form of a look-up table, this calibration can be carried out efficiently. Preferably, such a look-up table will provide a multiplicative factor to be applied to the determined size measure in order to compensate the effect of a different body temperature of the patient. The main advantage resulting from said calibration is that the accuracy of the derived information on the vital state of the patient can be further increased.

A further possibility to calibrate the measurements is by measuring the distance between two observed blood vessels (which should be fairly constant irrespective of temp etc.) over time and comparing this to a pre-determined 'calibrated' measurement in order to calibrate the instrument, so that it can then be used to more accurately measure the size of an individual blood vessel that is captured. The evaluation unit may thus be configured to measure over time a distance between two blood vessels in the area of interest, to derive a distance of the detection unit relative to the area of interest therefrom and to correct the determined size measure for the blood vessel based on the derived distance of the detection unit relative to the area of interest.

In another embodiment, the therapy system further comprises a feedback unit for providing the information on the vital state of the patient to the patient and/or to a physician.

Such a feedback unit may be represented by a display, a wired or wireless data interface, a haptic interface, an acoustic interface, etc.. This feedback unit may be arranged at the patient interface or remotely thereto. Via this feedback unit, the derived information on the vital state of the patient, i.e. in particular one or more vital parameters or trend parameters is provided to the patient and/or to the physician of the patient in order to provide feedback on the development of the vital state of the patient to modify the therapy or to modify the behavior of the patient. Providing the information on the vital state to the patient himself may particularly result in an increased compliance of the patient, as the patient obtains immediate feedback on how his therapy affects his vital state. Providing the information on the vital state of the patient to the physician allows the physician to obtain information on how the prescribed treatment takes effect and/or modify the prescribed therapy. Depending on the application, it may be possible to provide the information on the vital state of the patient in a form that allows the patient or the physician to immediately obtain information on a parameter being relevant for a currently provided treatment.

In yet another embodiment, the therapy system further comprises a database interface for communicating with a database including information on a vital state of reference patients, wherein the evaluation unit is configured to derive information on the vital state of the patient including at least one comparison parameter being indicative of a relation between the vital state of the patient and the vital state of the reference patients.

This interface may particularly be represented by a wired or wireless communication interface for connecting the patient interface presented herein with a database (e.g. an online database) including information on reference patients. This information allows the evaluation unit to base the evaluation of the development of the determined size measure over time also on a comparison of the patient with other patients (reference patients) that suffer from a comparable disease and/or receive a comparable treatment. This may then allow providing a comparison parameter, such as a parameter that represents a relation of the blood pressure of the patient with the blood pressure of other patients, or the like. Therefrom, the patient and/or the physician can obtain information on how the vital state of the patient compares to the vital state of other patients. This may further improve the compliance of the patient and may allow the physician to obtain a reference when working out an optimal therapy for the patient. It shall be understood that the database itself may also be part of the herein presented patient interface, i.e. included therein or attached thereto.

In yet another embodiment, the patient interface further comprises an illumination unit for illuminating the skin area of the patient. An additional illumination source can help to improve the image quality of the generated image signal. In a particular embodiment, the illumination can be configured to illuminate the skin area of the patient with light of a specific wavelength. For instance, red light travels further into the skin than blue light. Thus, an illumination wavelength may be selected based on the depth of the blood vessels to be imaged.

As mentioned above, the present invention does not only pertain to the whole therapy system, but also to a therapy device that may form part of said system. The therapy device preferably comprises a pressure generator for generating a pressurized flow of breathable gas for delivery to a patient interface; a data interface for receiving an image signal from a detection unit that is configured to detect reflected light from a skin area of the patient and to generate the image signal from the detected light; a processing unit for processing the image signal; and an evaluation unit for deriving information on a vital state of the patient based on an evaluation of a development of the image signal over time.

According to a preferred embodiment, said therapy device further comprises a control unit that is configured to adjust the settings of the pressure generator based on the derived information on the vital state of the patient.

It shall be understood that the described embodiments are also possible in various combinations of the appended claims even if not explicitly described herein or indicated by means of the claim dependencies.

### BRIEF DESCRIPTION OF THE DRAWINGS

These and other aspects of the invention will be apparent from and elucidated with reference to the embodiment(s) described hereinafter. In the following drawings
Fig. 1 schematically illustrates a patient receiving treatment by means of a therapy system according to an embodiment of the present invention;
Fig. 2 shows an illustration of a patient interface for delivering a flow of breathable gas to a patient according to an embodiment of the present invention;
Fig. 3 shows a schematic block diagram of the different units of the therapy system according to an embodiment of the present invention;
Fig. 4 shows a schematic block diagram of further units of the therapy system according to a further embodiment of the present invention; and
Fig. 5 shows a schematic illustration of a method for obtaining information on a vital state of a patient according to an embodiment of the present invention.

### DETAILED DESCRIPTION OF THE INVENTION

As described above, OSA is a condition whereby a person's airways become blocked due to the collapse of soft tissue at the back of the throat. The result of this blockage is that the oxygenation of the person's blood reduces. The severity of the condition can be measured by means of the AHI. The AHI is a number equal to the number of apnea events per hour. The exact definition of an apnea event varies, but a typical definition is that the breathing has stopped for ten or more seconds and an associated reduction in blood oxygenation is observed. Such events may have an effect on the blood pressure because a drop in blood oxygenation can result in the physical reaction that the blood vessels are constricted in order to prioritize the flow of blood to the heart and brain. For instance, it is shown in Somers et al., Sympathetic Neural Mechanisms in Obstructive Sleep Apnea, J. Clin. Invest., Volume 96, October 1995, that the rate of flow of blood can be correlated to the AHI. Other mechanisms that affect the blood pressure are, e.g., the consumption of alcohol or smoking.

The present invention allows remotely deriving information on the vital state of the patient by means of an image sensor that is integrated into a patient interface. One particular embodiment allows evaluating changes in the size of a blood vessel over time by evaluating the images or image sequences generated with the image sensor and deriving the vital state of the patient therefrom. For instance, a parameter being indicative of an AHI or of a blood pressure of the patient can be extracted from the taken images. Current techniques for tracking the AHI include, apart from polysomnography (which usually requires the patient to be hospitalized), also Neural Network Analysis, Nasal Pressure Recording and Nasal Air Flow Monitoring. These methods have the disadvantages that the patient's sleep needs to be disturbed or the patient needs to be woken up in order to carry out a measurement. Also, some measurement procedures, such as polysomnography, need to be carried out in a hospital and are therefore expensive. The present invention may allow tracking the AHI, pulse rate, blood oxygenation, blood pressure and/or blood pressure changes during one or multiple sleep sessions and comparing the determined values to previously determined values for the same patient or for other patients suffering from comparable diseases without requiring the patient to wake up or to perform a dedicated or disturbing measurement procedure. This will be explained in the following by means of a particular embodiment with reference to the accompanying drawings.

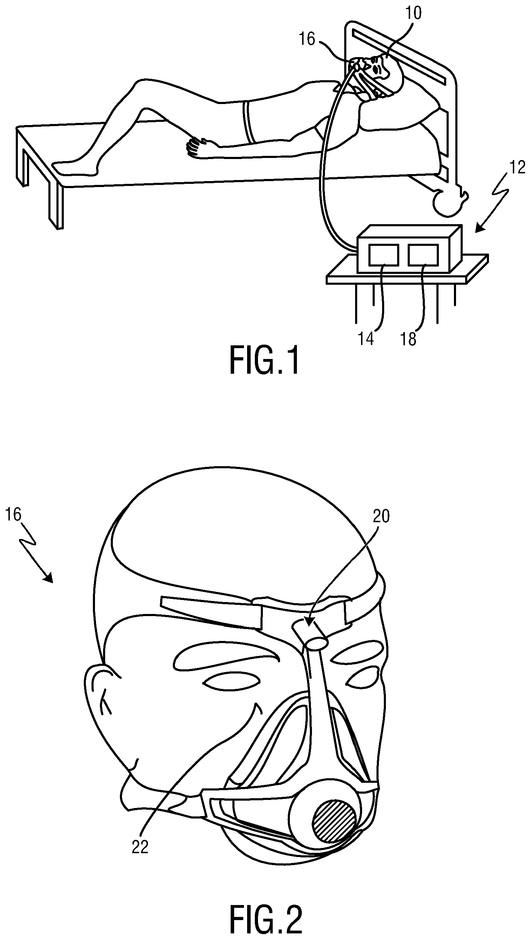

Fig. 1 shows a patient 10 receiving OSA treatment by means of therapy system according to an embodiment of the present invention. The therapy system in this embodiment includes a therapy device 12 and a patient interface 16. The therapy device 12 comprises a pressure generator 14 including some kind of ventilator for generating a pressurized flow of breathable gas and a control unit 18 for manually or automatically adjusting the settings of the pressure generator 14. The pressurized flow of breathable gas generated by the pressure generator 14 is provided to the patient 10 via the patient interface 16. The patient interface 16 is preferably connected to the pressure generator 14 by means of a flexible hose. The patient interface 16 may, e.g., be represented by a nasal mask fitting over the nose and delivering gas through the nasal passages, an oral mask, fitting over the mouth and delivering gas through the mouth or a full-face mask fitting over both the nose and the mouth. The patient interface is usually fixed to the patient's head using some kind of headgear.

The patient interface 16 in the shown embodiment further comprises means 20 for deriving information on a vital state of the patient 10. At least some of the different components of these means 20 may be integrated in a camera or image sensor that is attached to the patient interface 16, as this is shown in Fig. 2. Fig. 2 illustrates an embodiment of a patient interface 16 including a camera 20 that is directed to a skin area 22 in the face of the patient 10. The skin area 22 observed by the camera 20 may be e.g. represented by an area around the nose of the patient 10. However, it is to be understood that the embodiment illustrated in Fig. 2 only represents one example for a possible camera position. Other embodiments may include a camera 20 being attached to other parts of the patient interface 16 or being partly or entirely integrated into the patient interface 16 and directed to other skin areas 22 in the patient's face, e.g. to the forehead, between the eyes or around the mouth.

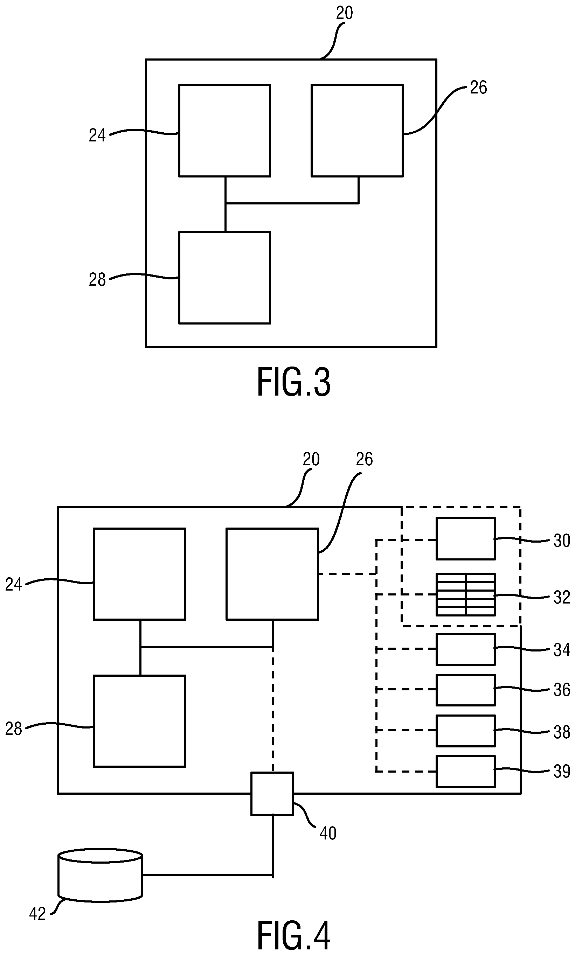

Fig. 3 schematically illustrates possible components of the camera 20. As illustrated in Fig. 3, the camera 20 comprises a detection unit 24, a processing unit 26 and an evaluation unit 28. The units 24, 26 and 28 may be housed in a single housing, e.g. in the camera housing illustrated in Fig. 2. However, it may also be possible that some units are spatially separated from the others and housed in separate housings. It shall be furthermore noted that the detection unit 24, the processing unit 26 and the evaluation unit 28 may either be partly or fully combined or realized as separate entities. The processing unit 26 and the evaluation unit 28 may be hardware and/or software-implemented.

The detection unit 24 may correspond to the camera 20 or, more precisely, to the image sensor of the camera 20. The detection unit can 24, e.g., correspond to a CCD or CMOS sensor, which allows capturing brightness values for an array of photosensitive elements (pixels). Usually, different photosensitive elements are sensitive in different frequency bands, i.e. correspond to different colors. The detection unit 24 detects reflected light from the skin area 22 of interest. This skin area 22 may thereto additionally be illuminated by means of a dedicated light source (not explicitly shown) emitting light of at least one wavelength interval, or may be illuminated by ambient light. The detection unit 24 is preferably mounted to the patient interface 16 by means of a mounting structure, such that a fixed distance to the skin area 22 can be maintained. Alternatively, it is also possible that the detection unit 24 is in direct contact with the skin area 22 of the patient 10. However, it is in all cases preferred that the detection unit 24 is part of the patient interface 16, whereas the processing unit 26 and the evaluation unit 28 do not have to be arranged at the patient interface 16. Both the processing unit 26 and the evaluation unit 28 may also be arranged remote from the patient interface 16, e.g. form part of the therapy device 12, a remote computer or a mobile device. In the latter mentioned case, the processing unit 26 and the evaluation unit 28 may be connected to the detection unit 24 of the patient interface via a wireless connection. The patient interface 16 may thereto comprise a suitable communication interface for wireless communication with the processing unit 26 and the evaluation unit 28.

The basic principal underlying the present invention is to observe propagating blood as the heart pumps by extracting visible changes in the blood vessels. With each heartbeat there is a 'surge' travelling through the blood vessels. The propagation of the lead part of this surge can be extracted from the image signal by means of image processing algorithms. From evaluating one or more of such surges and determining their (relative) intensity, e.g., a rate of flow of blood or a heart rate can be derived.

For this, the processing unit 26 receives the image signal from the detection unit 24 processes the image signal and generates an image sequence therefrom. The processing unit 26 furthermore identifies one or more blood vessels of the patient 10 within the skin area 22. The processing unit 26 preferably applies an image processing algorithm for detecting and identifying a blood vessel within this image sequence. The identification of a blood vessel includes determining whether the image sequence includes a blood vessel at all and assigning a unique identification to each of the observed blood vessels. Due to the known pulsating movement of the blood vessels, the blood vessels are relatively easy to detect within the image sequence. Image processing algorithms, such as edge detection or others, can be used. An example for an edge detection algorithm is Canny edge detection. Blood vessels could also be identified in the image using various object recognition algorithms, e.g. by breaking the image into components and comparing these components to 'known' examples, i.e. previously recorded images that have been saved to a database, i.e. by looking for similarities.

After identifying the one or more blood vessels, the processing unit 26 determines a size measure for at least one of the one or more identified blood vessels. Such a size measure preferably includes a diameter (width) or an average diameter in one or more sections of the blood vessel. It shall be noted that this size measure determination is of course based on an approximation. The diameter may thereby be estimated at set distances along its length. This is preferably done over a group of heartbeats (during which time the blood vessel expands and contracts) and at a frequency which is much greater than the frequency of the heartbeat. The frames of the image sequence are thus captured at a frequency (frame rate) substantially higher than the heart rate of the patient 10. The generated image sequence preferably covers a time period including at least a group of heartbeats.

The evaluation unit 28 then evaluates the diameter of the examined blood vessel changes over time and derives a vital state of the patient 10 therefrom. The evaluation unit 28 may be particularly configured to analyze how the diameter of the blood vessel changes during a heartbeat cycle of the patient 10. Furthermore, the evaluation unit 28 may be configured to evaluate how the diameter of the blood vessel develops during multiple heartbeat cycles, e.g. to identify a tendency or trend. Such a trend may cover a period of a few heartbeat cycles or also a longer period such as a sleep cycle or multiple sleep cycles.

The consideration of the blood vessel diameter change at one or multiple sections along the length of the blood vessel allows estimating several vital parameters, such as the heart rate, the blood flow rate, a change in blood pressure or even an indication about the blood pressure itself.

The heart rate can be estimated by analyzing the frequency of changes in the size of the blood vessel. A blood vessel regularly changes its size caused by the pulsating blood. The frequency of these changes can be extracted and used as an indication of the heart rate of the patient 10. This may be e.g. done by measuring the time difference between two subsequent surges of the blood vessel. This can be measured by evaluating the diameter change at a distinctive point of the blood vessel over time and measuring the time between two maxima or two minima of the diameter at said point. It shall be noted that the maximum diameter denotes the diameter of the blood vessel when a surge arrives at said point and the vessel is expanded; and the minimum diameter denotes the diameter of the blood vessel when no surge is at said point and the vessel is contracted. Of course, several measurements between subsequent maxima or minima may be performed in order to make the approximation more robust.

The approximated blood flow rate may be determined by calculating the average diameter over time and estimating the velocity of the blood. The velocity of the blood may be estimated by measuring the velocity of the surge travelling through the blood vessel during each heartbeat. The velocity of the surge can be determined by evaluating the diameter change of the blood vessel at two points (having known distances from each other) along the length of the blood vessel and stopping the time the diameter maximum (caused by the surge) travels from the first point to the second.

The change of the minimum, maximum and/or average blood vessel diameter over time may also give an indication of the blood pressure change of the patient. The increase of the minimum blood vessel diameter and/or the increase of the difference between the maximum and minimum blood vessel diameter correlate with a blood pressure increase. Thus, conclusions regarding blood pressure changes can be drawn from the diameter changes of the blood vessel.

The evaluation unit 28 may even be configured to give an estimation of the blood pressure itself by evaluating the diameter changes of the blood vessel. The blood flow rate, which can be estimated as outlined above, is usually defined as a pressure difference divided by a resistance, as e.g. described online in "Pressure and Blood Flow" (http://math.arizona.edu/~maw1999/blood/pressure.html). The resistance of a blood vessel in the circulatory system is related to the vessel radius (the larger the radius, the lower the resistance), vessel length (the longer the vessel, the higher the resistance), blood viscosity, as well as the smoothness of the blood vessel walls. Smoothness may be reduced by the buildup of fatty deposits on the arterial walls. Vessel length will not change if always the same blood vessel is imaged and the width of the vessel can be monitored (extracted size measure). Fatty deposits will build up over a timescale longer than the observation. Hence, these factors can be either corrected or omitted.

Thus, a relationship between flow rate and blood pressure can be exploited. Therefore, the derived information may also include a vital parameter being indicative of a blood pressure of the patient.

Furthermore, some useful information may be obtained from the blood flow rate. For instance, in Urbano et al., Impaired Cerebral Autoregulation in Obstructive Sleep Apnea, J Appl Physiol, 2008 as well as in Netzer et al, Blood Flow of the Middle Cerebral Artery With Sleep-Disordered Breathing, Stroke, 1998, the blood flow rate in the middle cerebral artery is measured, which is matched by a higher blood flow in the face.

Still further, another vital parameter may also be indicative of an AHI of the patient 10. As outlined above, apnea and hypopnea events have an effect on the blood pressure of a person. Thus, by monitoring the blood flow rate also information on the AHI can be derived.

In addition to these vital parameters that are usually derived for short time period, such as a time period covering a few heartbeat cycles of the patient, it is also possible to monitor a long term development of these measures. For this, the evaluation unit 28 can be configured to derive at least one trend parameter. Such a trend parameter basically represents a development of a vital parameter during one sleep cycle (sometimes also referred to as sleep session) or over a period including multiple sleep cycles of the patient 10 (e.g. one week). The long term parameter may also be indicative of an average or a minimum/maximum value of one of the vital parameters. For instance, it may be of interest to monitor the AHI or the blood pressure of the patient 10 during a time interval, such as one week or one month etc.

Instead of evaluating a size measure of a blood vessel over time, the evaluation unit 28 may also be configured to evaluate a red color content of the image pixels of the image sequence in the area of interest 22 and to evaluate a development of said red color content of the image pixels of the image sequence in said area 22 over time in order to derive the information in the vital state of the patient. For example, it is possible to examine over time the average 'redness', or the minimum and/or maximum of the red color content, or a percentage of the red color content over a certain level of red value. In particular by evaluating over time the a percentage of the red color content over a certain level of red value of the pixels in the area of interest 22, the evaluation unit 28 may be configured to derive therefrom an estimate about the heart rate, a change of rate of flow of blood over time and/or a change of blood pressure over time. Such an estimate may be based on the consideration that an increase in the red color content over time may result from an increased blood flow and/or blood pressure over time. By evaluating the change of the red color content over time, the pulsation of blood may also be seen, such that the heart rate may be extracted.

The functionalities of the processing unit 26 and the evaluation unit 28 may be carried out partly or entirely in a microprocessor, e.g. an integrated circuit (IC) or an application specific integrated circuit (ASIC). The functionality may partly or entirely be implemented in hard- and/or in software. therapy system may also include peripheral equipment such as storage means, wiring or mechanical components for mechanically fixing the different parts.

In a preferred embodiment, Eulerian video magnification or another image magnification technique is applied to the image signal prior to identifying a blood vessel or at least prior to determining its size. This technique can facilitate the blood vessel identification and size determination. The processing unit 26 is preferably configured to carry out the necessary operations. Video magnification techniques herein refer to techniques that allow amplifying subtle changes in a video sequence. Usually, the pulsating blood will only cause a small movement of the blood vessel. In order to be able to detect such a small movement the image signal needs to be amplified. Usually, parts of the image (signal portion) that show strong motion components are identified and the motion is intensified by modifying the values of single pixel as lined out in the paper on Eulerian video magnification cited above. The outlined approach includes a spectral analysis and decomposition as well as the application of different filtering approaches. By applying video magnification to the image signal, the identification of a blood vessel as well as the determination of the size measure becomes more reliable. Eulerian video magnification may also be used to amplify the red color content within the image sequence, which may facilitate the above mentioned option in which the information in the vital state of the patient is derived based on an evaluation of the red color content change over time of certain pixels in the derived image sequence.

Fig. 4 schematically illustrates a block diagram of patient interface 16, in particular of the electronic components of the camera 20 and image processing units, according to a further refinement. Apart from the detection unit 24, the processing unit 26 and the evaluation unit 28, there are illustrated various other components that may be included in embodiments of the present invention. Some of the shown components are to be considered optional and may be integrated in the patient interface 16 or remote therefrom.

Fig. 4 illustrates that a temperature sensor 30 may provide a reading of the body temperature of the patient 10 and/or the ambient temperature to the processing unit 26. Based on this body temperature and/or ambient temperature, the processing unit 26 can calibrate the determined size measure for the blood vessel in order to compensate for temperature-related effects. This temperature sensor 30 may be connected wirelessly or hardwired and may be integrated with the patient interface 10 or attached to the patient 10 at another position remote from the patient interface 16. The calibration may be based on a predetermined calibration function.

Alternatively to a calibration function, there may be comprised a look-up table 32 that includes predetermined calibration values for different body temperatures. Thus, it becomes possible to directly determine a calibration value based on the look-up table 32 for the current reading of the temperature sensor 30. This look-up table 32 may be integrated within the temperature sensor 30 or may be provided to the processing unit 26 in the form of a database or as a computer-readable file.

In yet another embodiment, it may also be possible that an ambient temperature is measured by means of an ambient temperature sensor (being mounted to the patient interface or communicating its sensor readings to an interface) and that the calibration is performed based on this ambient temperature.

There is further illustrated an orientation sensor 34 for providing an orientation parameter being indicative of an orientation of the detection unit 24 relative to the skin area 22 of the patient 10. Such an orientation sensor 34 may include an inertial sensor, such as an acceleration sensor or a gyroscope sensor, or a proximity sensor. The orientation sensor 34 provides a sensor signal (orientation parameter) that can be used to estimate the current alignment of the detection unit 24 relative the skin area 22. If, e.g., the patient 10 moves during the night and the position of the patient interface 16 relative to his face is changed, it may be required that the processing unit 26 is provided with a reading of the orientation of the detection unit 24 relative to the skin area 22. The orientation sensor 34 can provide such a reading. The processing unit 26 can then compensate for the change in the camera position prior to identifying a blood vessel and determining a size measure. This ensures that always the same skin area 22 is examined over time.

Still further, the patient interface 16 may comprise an actuation unit 36 for adjusting the alignment of the detection unit 24 relative to the skin area 22 of the patient 10. Such an actuation unit 36 may, e.g. be represented by an electronic motor or other electric actuator that allows changing the orientation and/or relative position of the detection unit 24. This actuation unit 36 may adjust the alignment based on the output of the orientation unit 34 and/or on a result of an image matching algorithm (e.g. image stitching or feature detection), which is applied to the detected image signal in the processing unit 26. The actuation unit 36 may also be used to assure a constant distance between the detection unit 24 and the skin area 22 of interest in order to facilitate the identification of a blood vessel of the patient 10. Alternatively, the distance between the detection unit 24 and the skin area 22 may also be calibrated based on observed distances in the image. For instance, the blood vessels may get larger/smaller but two blood vessels will not change their relative location, such that the distance between their centers remains constant. Therefore, it is possible to use a first measurement by means of a calibrated system to calibrate the patient interface mounted system of the present invention. For instance, the system can be calibrated by measuring the distance between two blood vessels and using this information to correct a size measure that has been determined after the position of the patient interface has been changed.

Still further, the therapy system may comprise a feedback unit 38 for providing feedback to the patient and/or to a physician. This feedback includes the determined information on the vital state of the patient 10. The feedback unit 38 may, e.g., be represented by a display being integrated with the patient interface 16, which displays at least one parameter indicative of the vital state of the patient 10. Preferably, however, the feedback unit 38 may be represented by a communication interface, such as a wireless transceiver, which allows transmitting the determined information on the vital state of the patient 10 to a mobile information device (smartphone or tablet or the like) or to a personal computer. These devices may then be configured to provide the derived information on the vital state of the patient 10 in an appropriate form. Thereby, the communication may either be uni- or bidirectional. A bidirectional communication may also allow the patient and/or the physician to configure the output of the evaluation unit 28.

In another embodiment, there may be comprised an illumination unit 39. Such an illumination unit 39 can, e.g., be represented by a LED or other light source emitting light of a broad spectrum or of a dedicated wavelength. The emitted light is directed to the skin area 22 of the patient 10. The detection unit may then detect reflected light that mainly corresponds to the light of the illumination unit 39. Such an illumination can result in an improved quality of the generated image signal.

Still further, the therapy system may comprise a database interface 40, which is configured to communicate with a database 42 that includes information on a vital state of reference patients. This information on a vital state of reference patients may refer to information that has been collected by means of comparable patient interfaces. Such reference patients allow an evaluation of how the vital state of the patient 10 is in comparison to the vital state of reference patients. For instance, a patient suffering from OSA may find that other patients have a lower AHI and a lower blood pressure. The patient 10 may take this comparison as a reason to question whether he has chosen the appropriate settings for his pressure support system. If information of reference patients is available, the evaluation unit 28 may be further configured to determine a comparison parameter. Such a comparison parameter may include a percentage value representative of a relation of the AHI of the patient with the AHI of the reference patients or an absolute value representing a ranking of the patient in comparison to the reference patients with regard to his vital state (blood pressure etc.).

In yet another embodiment, there may further be comprised a user interface for providing a possibility for the patient to enter patient information. Such patient information may, e.g., include information about lifestyle activities that may have an effect on the measurements taken (sport, alcohol/cigarette consumption, etc.). This information can then also be used as an educative tool to show how these lifestyle decisions have affected the patient's AHI. It may also be possible that the control of various settings on the patient's PAP machine is further based on this information.

In another aspect of the present invention the processing unit 26, the evaluation unit 26 and some of the other above-mentioned components of the therapy system may be integrated into the therapy device 12 as illustrated in Fig. 1. The derived information on the vital state of the patient 10 can then also be used to directly control a pressure generator 14, i.e. to provide a closed-loop control of a pressure support system. Examples of settings of the pressure generator 14 that may be changed include the airflow rate and pressure, which may then have an effect on the patients AHI. Changes of these settings would be made when the derived information on the vital state of the patient indicates that the patient's AHI level was too high. Another example could be that a patient 10 having a high blood pressure may need to be provided with another gas composition than a patient 10 with a lower blood pressure.

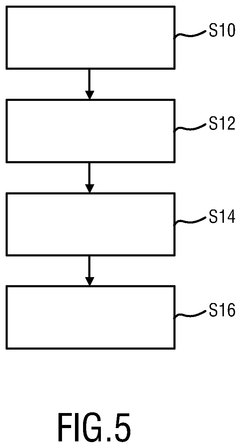

In Fig. 5 a method for obtaining information on a vital state of a patient 10 is schematically illustrated. This method includes detecting (S10) reflected light from a skin area 22 of the patient 10 and generating (S12) an image signal from the detected light by means of a detection unit 24 that is comprised in a patient interface 16 for delivering a flow of breathable gas to the patient 10; processing (S 14) the image signal; and evaluating (S16) the image signal for deriving information on the vital state of the patient 10 based on an evaluation of a development of the image signal over time. Such a method may particularly be carried out on a computer or microprocessor being included in a patient interface and being in communication with a camera or image sensor.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. A therapy system comprising:
a patient interface (16) for delivering a flow of breathable gas to a patient (10), wherein the patient interface (16) comprises a detection unit (24) for detecting reflected light from a skin area (22) of the patient (10);
a processing unit (26) for processing the image signal; and
an evaluation unit (28) for deriving information on a vital state of the patient (10) based on an evaluation of a development of the image signal over time;
**characterized in that** the detection unit (24) is configured to generate an image signal from the detected light and **in that** the processing unit (26) is configured to identify a blood vessel of the patient (10) within the skin area (22) and to determine a size measure for the blood vessel, and wherein the evaluation unit (28) is configured to evaluate a development of the determined size measure over time to derive information on the vital state of the patient (10).

2. The therapy system according to claim 1, wherein the processing unit (26) is configured to amplify a signal portion of the image signal being indicative of at least one of a movement and a change in color in the skin area (22).

3. The therapy system according to claim 1, wherein the evaluation unit (28) is configured to derive information on the vital state of the patient (10) including at least one vital parameter being indicative of:
a minimum, maximum or average diameter of the blood vessel during at least one heartbeat of the patient (10);
a blood pressure and/or change of the blood pressure over time of the patient (10);
a heart rate of the patient (10);
an apnea-hypopnea-index of the patient (10);
a rate of flow of blood of the patient (10); and
a variation of the rate of flow of blood.

4. The therapy system according to claim 1, wherein the processing unit is configured to derive from the image signal an image sequence including at least one image of the skin area, wherein the therapy system further comprises a storage unit for storing previously recorded image sequences of the skin area, and wherein the evaluation unit (28) is configured to derive the information on the vital state of the patient by comparing the at least one image of the skin area with at least one image of the previously recorded image sequences of the skin area.

5. The therapy system according to claim 4, wherein the processing unit is configured to track the skin area within the image sequence and/or the previously recorded image sequences by applying an image matching algorithm.

6. The therapy system according to claim 1, wherein the patient interface (16) further comprises an orientation sensor (34) for measuring an orientation of the detection unit (24) relative to the skin area (22) of the patient (10).

7. The therapy system according to claim 6, wherein the patient interface (16) further comprises an actuator (36) for adjusting a position and/or alignment of the detection unit (24) relative to the skin area (22) of the patient (10) based on the orientation measured by the orientation sensor (34).

8. The therapy system according to claim 1, further comprising a temperature sensor for measuring a body temperature and/or an ambient temperature, wherein the evaluation unit (28) is configured to correct the evaluation of the development of the image signal based on the body temperature and/or the ambient temperature.

9. The therapy system according to claim 1, further comprising a database interface (40) for communicating with a database (42) including information on a vital state of reference patients;
wherein the evaluation unit (28) is configured to derive information on the vital state of the patient (10) including at least one comparison parameter being indicative of a relation of the vital state of the patient (10) to the vital state of the reference patients.

10. Therapy device (12) comprising:
a pressure generator (14) for generating a pressurized flow of breathable gas for delivery to a patient interface (16);
a data interface for receiving an image signal from a detection unit (24) that is configured to detect reflected light from a skin area (22) of the patient (10);
a processing unit (26) for processing the image signal; and
an evaluation unit (28) for deriving information on a vital state of the patient (10) based on an evaluation of a development of the image signal over time; **characterised in that** the detection unit (24) is adapted to generate the image signal from the detected light and wherein
the processing unit (26) is configured to identify a blood vessel of the patient (10) within the skin area (22) and to determine a size measure for the blood vessel, and wherein the evaluation unit (28) is configured to evaluate a development of the determined size measure over time to derive information on the vital state of the patient (10).

11. Therapy device according to claim 10, further comprising a control unit (18) that is configured to adjust the settings of the pressure generator (14) based on the derived information on the vital state of the patient (10).

12. Method for obtaining information on a vital state of a patient (10), comprising:
detecting (S10) reflected light from a skin area (22) of the patient (10) and generating (S12) an image signal from the detected light by means of a detection unit (24) that is comprised in a patient interface (16) for delivering a flow of breathable gas to the patient (10);
processing (S14) the image signal; and
evaluating (S16) the image signal for deriving information on the vital state of the patient (10) based on an evaluation of a development of the image signal over time; wherein
the processing (S14) includes identifying a blood vessel of the patient (10) within the skin area (22) and determining a size measure for the blood vessel, and wherein the evaluating (S16) includes evaluating a development of the determined size measure over time.

13. Computer program comprising program code means for causing a computer to carry out the steps of the method as claimed in claim 12 when said computer program is carried out on the computer.

## Patentansprüche

1. Therapiesystem, umfassend:
eine Patientenschnittstelle (16) zum Zuführen einer Atemgasströmung zu einem Patienten (10), wobei die Patientenschnittstelle (16) eine Detektionseinheit (24) umfasst, um von einem Hautbereich (22) des Patienten (10) reflektiertes Licht zu detektieren;
eine Verarbeitungseinheit (26) zum Verarbeiten des Bildsignals; und
eine Evaluierungseinheit (28) zum Ableiten von Informationen über einen Vitalzustand des Patienten (10) basierend auf einer Evaluierung einer Entwicklung des Bildsignals über die Zeit;
**dadurch gekennzeichnet, dass** die Detektionseinheit (24) konfiguriert ist, um ein Bildsignal anhand des detektierten Lichts zu erzeugen, und dadurch, dass die Verarbeitungseinheit (26) konfiguriert ist, um ein Blutgefäß des Patienten (10) innerhalb des Hautbereichs (22) zu identifizieren und ein Größenmaß für das Blutgefäß zu ermitteln, und wobei die Evaluierungseinheit (28) konfiguriert ist, um eine Entwicklung des ermittelten Größenmaßes über die Zeit zu evaluieren, um Informationen über den Vitalzustand des Patienten (10) abzuleiten.

2. Therapiesystem nach Anspruch 1, wobei die Verarbeitungseinheit (26) konfiguriert ist, um einen Signalabschnitt des Bildsignals zu verstärken, der mindestens entweder eine Bewegung oder eine farbliche Veränderung im Hautbereich (22) angibt.

3. Therapiesystem nach Anspruch 1, wobei die Evaluierungseinheit (28) konfiguriert ist, um Informationen über den Vitalzustand des Patienten (10) einschließlich mindestens eines Vitalparameters abzuleiten, der Folgendes angibt:
einen minimalen, maximalen oder durchschnittlichen Durchmesser des Blutgefäßes während mindestens eines Herzschlags des Patienten (10);
einen Blutdruck und/oder einer Änderung des Blutdrucks des Patienten (10) über die Zeit;
eine Herzfrequenz des Patienten (10);
einen Apnoe-Hypopnoe-Index des Patienten (10);
eine Blutströmungsrate des Patienten (10); und
eine Variation der Blutströmungsrate.

4. Therapiesystem nach Anspruch 1, wobei die Verarbeitungseinheit konfiguriert ist, um eine Bildsequenz einschließlich mindestens eines Bilds des Hautbereichs von dem Bildsignal abzuleiten, wobei das Therapiesystem weiterhin eine Speichereinheit zum Speichern von zuvor aufgenommenen Bildsequenzen des Hautbereichs umfasst, und wobei die Evaluierungseinheit (28) konfiguriert ist, um die Informationen über den Vitalzustand des Patienten abzuleiten, indem sie das mindestens eine Bild des Hautbereichs mit mindestens einem Bild der zuvor aufgenommenen Bildsequenzen des Hautbereichs vergleicht.

5. Therapiesystem nach Anspruch 4, wobei die Verarbeitungseinheit konfiguriert ist, um den Hautbereich in der Bildsequenz und/oder den zuvor aufgenommenen Bildsequenzen zu verfolgen, indem sie einen Bildabgleichalgorithmus anwendet.

6. Therapiesystem nach Anspruch 1, wobei die Patientenschnittstelle (16) weiterhin einen Orientierungssensor (34) zum Messen einer Orientierung der Detektionseinheit (24) in Bezug auf den Hautbereich (22) des Patienten (10) umfasst.

7. Therapiesystem nach Anspruch 6, wobei die Patientenschnittstelle (16) weiterhin ein Betätigungselement (36) umfasst, um eine Position und/oder Ausrichtung der Detektionseinheit (24) in Bezug auf den Hautbereich (22) des Patienten (10) basierend auf der durch den Orientierungssensor (34) gemessenen Orientierung zu justieren.

8. Therapiesystem nach Anspruch 1, weiterhin umfassend einen Temperatursensor zum Messen einer Körpertemperatur und/oder einer Umgebungstemperatur, wobei die Evaluierungseinheit (28) konfiguriert ist, um die Evaluierung der Entwicklung des Bildsignals basierend auf der Körpertemperatur und/oder der Umgebungstemperatur zu korrigieren.

9. Therapiesystem nach Anspruch 1, weiterhin umfassend eine Datenbankschnittstelle (40) zum Kommunizieren mit einer Datenbank (42) mit Informationen über einen Vitalzustand von Referenzpatienten;
wobei die Evaluierungseinheit (28) konfiguriert ist, um Informationen über den Vitalzustand des Patienten (10) einschließlich mindestens eines Vergleichsparameters abzuleiten, der eine Beziehung des Vitalzustands des Patienten (10) zu dem Vitalzustand der Referenzpatienten angibt.

10. Therapievorrichtung (12), umfassend:
einen Druckgenerator (14) zum Erzeugen einer unter Druck stehenden Atemgasströmung zur Zuführung zu einer Patientenschnittstelle (16);
eine Datenschnittstelle zum Empfangen eines Bildsignals von einer Detektionseinheit (24), die konfiguriert ist, um von einem Hautbereich (22) des Patienten (10) reflektiertes Licht zu detektieren;
eine Verarbeitungseinheit (26) zum Verarbeiten des Bildsignals; und
eine Evaluierungseinheit (28) zum Ableiten von Informationen über einen Vitalzustand des Patienten (10) basierend auf einer Evaluierung einer Entwicklung des Bildsignals über die Zeit;
**dadurch gekennzeichnet, dass** die Detektionseinheit (24) dafür ausgelegt ist, das Bildsignal anhand des detektierten Lichts zu erzeugen, und wobei
die Verarbeitungseinheit (26) konfiguriert ist, um ein Blutgefäß des Patienten (10) innerhalb des Hautbereichs (22) zu identifizieren und ein Größenmaß für das Blutgefäß zu ermitteln, und wobei die Evaluierungseinheit (28) konfiguriert ist, um eine Entwicklung des ermittelten Größenmaßes über die Zeit zu evaluieren, um Informationen über den Vitalzustand des Patienten (10) abzuleiten.

11. Therapievorrichtung nach Anspruch 10, weiterhin umfassend eine Steuereinheit (18), die konfiguriert ist, um die Einstellungen des Druckgenerators (14) basierend auf den abgeleiteten Informationen über den Vitalzustand des Patienten (10) zu justieren.

12. Verfahren zum Erlangen von Informationen über einen Vitalzustand eines Patienten (10), umfassend:
das Detektieren (S10) von Licht, das von einem Hautbereich (22) des Patienten (10) reflektiert wurde, und das Erzeugen (S 12) eines Bildsignals anhand des detektierten Lichts mittels einer Detektionseinheit (24), die in einer Patientenschnittstelle (16) zum Zuführen einer Atemgasströmung an den Patienten (10) enthalten ist;
das Verarbeiten (S14) des Bildsignals; und
das Evaluieren (S16) des Bildsignals, um Informationen über einen Vitalzustand des Patienten (10) basierend auf einer Evaluierung einer Entwicklung des Bildsignals über die Zeit abzuleiten;
wobei
das Verarbeiten (S14) das Identifizieren eines Blutgefäßes des Patienten (10) innerhalb des Hautbereichs (22) und das Ermitteln eines Größenmaßes für das Blutgefäß umfasst, und wobei das Evaluieren (S16) das Evaluieren einer Entwicklung des ermittelten Größenmaßes über die Zeit umfasst.

13. Computerprogramm umfassend Programmcodemittel zum Veranlassen eines Computers, die Schritte des Verfahrens nach Anspruch 12 durchzuführen, wenn das genannte Computerprogramm auf dem Computer ausgeführt wird.

## Revendications

1. Système thérapeutique comprenant :
une interface patient (16) pour administrer un flux de gaz respirable à un patient (10), l'interface patient (16) comprenant une unité de détection (24) pour détecter la lumière réfléchie par une zone de peau (22) du patient (10) ;
une unité de traitement (26) pour traiter le signal d'image ; et
une unité d'évaluation (28) pour dériver des informations sur un état vital du patient (10) sur la base d'une évaluation d'un développement du signal d'image avec le temps ;
**caractérisé en ce que** l'unité de détection (24) est configurée pour générer un signal d'image à partir de la lumière détectée et **en ce que** l'unité de traitement (26) est configurée pour identifier un vaisseau sanguin du patient (10) au sein de la zone de peau (22) et pour déterminer une mesure de taille pour le vaisseau sanguin, et dans lequel l'unité d'évaluation (28) est configurée pour évaluer un développement de la mesure de taille déterminée avec le temps pour dériver des informations sur l'état vital du patient (10).

2. Système thérapeutique selon la revendication 1, dans lequel l'unité de traitement (26) est configurée pour amplifier une partie de signal du signal d'image indicative d'au moins l'un d'un mouvement et d'un changement de couleur dans la zone de peau (22).

3. Système thérapeutique selon la revendication 1, dans lequel l'unité d'évaluation (28) est configurée pour dériver des informations sur l'état vital du patient (10) comportant au moins un paramètre vital indicatif :
d'un diamètre minimum, maximum ou moyen du vaisseau sanguin pendant au moins un battement cardiaque du patient (10) ;
d'une pression sanguine et/ou d'un changement de la pression sanguine avec le temps du patient (10) ;
d'un rythme cardiaque du patient (10) ;
d'un index apnées-hypopnées du patient (10) ;
d'une vitesse d'écoulement de sang du patient (10) ; et
d'une variation de la vitesse d'écoulement du sang.

4. Système thérapeutique selon la revendication 1, dans lequel l'unité de traitement est configurée pour dériver du signal d'image une séquence d'images comportant au moins une image de la zone de peau, le système thérapeutique comprenant en outre une unité de stockage pour stocker les séquences d'images préalablement enregistrées de la zone de peau, et dans lequel l'unité d'évaluation (28) est configurée pour dériver les informations sur l'état vital du patient en comparant l'au moins une image de la zone de peau avec au moins une image des séquences d'images préalablement enregistrées de la zone de peau.

5. Système thérapeutique selon la revendication 4, dans lequel l'unité de traitement est configurée pour suivre la zone de peau au sein de la séquence d'images et/ou des séquences d'images préalablement enregistrées en appliquant un algorithme de correspondance d'image.

6. Système thérapeutique selon la revendication 1, dans lequel l'interface patient (16) comprend en outre un capteur d'orientation (34) pour mesurer une orientation de l'unité de détection (24) par rapport à la zone de peau (22) du patient (10).

7. Système thérapeutique selon la revendication 6, dans lequel l'interface patient (16) comprend en outre un actionneur (36) pour ajuster une position et/ou un alignement de l'unité de détection (24) par rapport à la zone de peau (22) du patient (10) sur la base de l'orientation mesurée par le capteur d'orientation (34).

8. Système thérapeutique selon la revendication 1, comprenant en outre un capteur de température pour mesurer une température corporelle et/ou une température ambiante, dans lequel l'unité d'évaluation (28) est configurée pour corriger l'évaluation du développement du signal d'image sur la base de la température corporelle et/ou de la température ambiante.

9. Système thérapeutique selon la revendication 1, comprenant en outre une interface de base de données (40) pour communiquer avec une base de données (42) comportant des informations sur un état vital de patients de référence ;
dans lequel l'unité d'évaluation (28) est configurée pour dériver des informations sur l'état vital du patient (10) comportant au moins un paramètre de comparaison indicatif d'une relation de l'état vital du patient (10) par rapport à l'état vital de patients de référence.

10. Dispositif thérapeutique (12) comprenant :
un générateur de pression (14) pour générer un flux sous pression de gaz respirable pour administration à une interface patient (16) ;
une interface de données pour recevoir un signal d'image d'une unité de détection (24) qui est configurée pour détecter la lumière réfléchie d'une zone de peau (22) du patient (10) ;
une unité de traitement (26) pour traiter le signal d'image ; et
une unité d'évaluation (28) pour dériver des informations sur un état vital du patient (10) sur la base d'une évaluation d'un développement du signal d'image avec le temps ;
**caractérisé en ce que** l'unité de détection (24) est configurée pour générer le signal d'image à partir de la lumière détectée et dans lequel
l'unité de traitement (26) est configurée pour identifier un vaisseau sanguin du patient (10) à l'intérieur de la zone de peau (22) et pour déterminer une mesure de taille pour le vaisseau sanguin, et dans lequel l'unité d'évaluation (28) est configurée pour évaluer un développement de la mesure de taille déterminée avec le temps pour dériver des informations sur l'état vital du patient (10).

11. Dispositif thérapeutique selon la revendication 10, comprenant en outre une unité de commande (18) qui est configurée pour ajuster les réglages du générateur de pression (14) sur la base des informations dérivées sur l'état vital du patient (10).

12. Procédé d'obtention d'informations sur un état vital d'un patient (10), comprenant :
la détection (S10) de la lumière réfléchie à partir d'une zone de peau (22) du patient (10) et la génération (S12) d'un signal d'image à partir de la lumière détectée au moyen d'une unité de détection (24) qui est comprise dans une interface patient (16) pour administrer un flux de gaz respirable au patient (10) ;
le traitement (S 14) du signal d'image ; et
l'évaluation (S16) du signal d'image pour dériver des informations sur l'état vital du patient (10) sur la base d'une évaluation d'un développement du signal d'image avec le temps ; dans lequel
le traitement (S14) comporte l'identification d'un vaisseau sanguin du patient (10) au sein de la zone de peau (22) et la détermination d'une mesure de taille pour le vaisseau sanguin, et dans lequel l'évaluation (S16) comporte l'évaluation d'un développement de la mesure de taille déterminée avec le temps.

13. Programme informatique comprenant des moyens de code de programme pour amener un ordinateur à exécuter les étapes du procédé selon la revendication 12 lorsque ledit programme informatique est exécuté sur l'ordinateur.
